# EUROPEAN PATENT SPECIFICATION

(11) **EP 1 991 586 B1**
(45) Date of publication and mention of the grant of the patent: **12.01.2011**
(21) Application number: 07716076.0
(22) Date of filing: 20.03.2007
(51) Int. Cl.: C07K 19/00, A61K 47/48, C07K 7/06

(54) **CHIMERIC CONSTRUCTS BETWEEN CANCER-HOMING PEPTIDES AND CELL-PENETRATING PEPTIDES COUPLED TO ANTICANCER DRUGS AND/OR DIAGNOSTIC AGENT/AGENTS**
CHIMÄRE KONSTRUKTE ZWISCHEN AUF KREBS ZIELENDEN PEPTIDEN UND ZELLPENETRIERENDEN PEPTIDEN GEKOPPELT AN ANTIKREBSMITTEL UND/ODER DIAGNOSTISCHE MITTEL
CONSTRUCTIONS CHIMÉRIQUES ENTRE PEPTIDES DE LOCALISATION DU CANCER ET PEPTIDES DE PÉNÉTRATION CELLULAIRE, COUPLÉES À DES MÉDICAMENTS ET/OU UN OU DES AGENTS DE DIAGNOSTIC ANTICANCER

(30) Priority: 20.03.2006 US 783396 P
(43) Date of publication of application: 19.11.2008
(73) Proprietor: Cepep III AB, 104 30 Stockholm (SE); Burnham Institute for Medical Research, La Jolla, CA 92037 (US)
(72) Inventor: LANGEL, Ülo, 113 37 Stockholm (SE); MYRBERG, Helena, 113 53 Stockholm (SE); MÄE, Maarja, 170 70 Solna (SE); RUOSLAHTI, Erkki, La Jolla, California 92037 (US); ZHANG, Lianglin, La Jolla, California 92037 (US)
(74) Representative: Nikolopoulou, Sofia
(86) International application number: PCT/SE2007/000268
(87) International publication number: WO 2007/108749

(56) References cited:
- WO-A-2005/094383
- WO-A1-2004/106370
- WO-A2-2004/108883
- US-A1- 2004 265 300
- US-A1- 2005 048 063
- M MÄE ET AL.: "Design of a tumor homing cell-penetrating peptide for drug delivery" INTERNATIONAL JOURNAL OF PEPTIDE RESEARCH AND THERAPEUTICS, vol. 15, no. 1, March 2009 (2009-03), pages 11-15, XP002516603 NL SPRINGER-VERLAG, DORDRECHT
- ELMQUIST A. ET AL.: 'VE-Cadherin-in-Derived Cell-Penetrating Peptide, pVEC, with Carrier Functions' EXPERIMENTAL CELL RESEARCH vol. 269, 2001, pages 237 - 244, XP003013956
- RUOSLAHTI E. ET AL.: 'Vascular Homing Peptides with Cell-Penetrating Properties' CURRENT PHARMACEUTICAL DESIGN vol. 11, 2005, pages 3655 - 3660, XP003013957

## Description

The present invention relates to chimeric constructs between the cancer-homing peptides CREKA (SEQ ID NO: 1), AREKA (SEQ ID NO:23), CREKA_{D} (SEQ ID NO:27) and a cyclic nonapeptide CPGPEGAGC (SEQ ID NO:2) and cell-penetrating peptides coupled to anticancer drugs and/or diagnostic agents for cancer treatment and/or diagnostic purposes. These constructs result in breast cancer cell homing and breast cancer cell penetration both *in vitro* and *in vivo*.

### Background

By definition, cell-penetrating peptides (CPP) consist of less than 30 amino acids and have a net positive charge ^{1; 2}. CPPs internalize in living animal cells *in vitro* ^{3; 4} and *in vivo* ^{5; 6} in both, endocytotic, or seemingly receptor- or energy- independent manner ^{7; 8} with subsequent re-evaluation of the translocation mechanisms for some CPPs ^{9; 10; 11}. There are several classes of CPPs with various origins, from totally protein-derived CPPs via chimeric CPPs to completely synthetic CPPs. The mechanism of internalization of CPPs is not yet characterized in detail, but the uptake seems to be different between the classes with a different element of endocytosis ^{12; 13}. *p*VEC (SEQ ID NO:6) is an 18-amino acid long cell-penetrating peptide derived from murine vascular endothelial cadherin (amino acids 615-632)^{14;15}.

The plasma membrane is impenetrable for most polar hydrophilic macromolecules as proteins and oligonucleotides. Those molecules internalize into animal cells mainly via endocytosis. Several endocytotic mechanisms have been described ¹⁶ among which the clathrin-dependent receptor-mediated endocytosis is used for internalization of ligand-receptor complexes ¹⁷. Most common procedures to deliver polar hydrophilic macromolecules into cells are electroporation and microinjection, but unfortunately the molecules can be delivered only in *in vitro* systems by these methods ^{18; 19}.

Blood vessels in normal individual tissues and different tumor tissues distinguish from each other due to the expression of different molecular markers ^{20; 21}. In tumors both blood and lymphatic vessels differ from normal vessels ²². Several peptides have been isolated by combining *ex vivo* and *in vivo* phage display for homing to tumors ^{23; 24; 25; 26}. Drug therapy for treating cancer is limited by a narrow therapeutic index. By coupling an anti-cancer drug to the homing motif it can be possible to direct the drug to the cancer cells ^{23; 27; 28}.

### Description of the invention

The present invention provides chimeric constructs between the cancer-homing peptides CREKA (SEQ ID No: 1), AREKA (SEQ ID NO:23), CREKA_{D} (SEQ ID NO:27) and a cyclic nonapeptide CPGPEGAGC (SEQ ID NO:2) and cell-penetrating peptides (CPPs) coupled to anticancer drugs and /or diagnostic agents for cancer treatment and/or diagnostic purposes.

By coupling a cancer-homing peptide to a CPP the construct will be able to internalize targeted cells. Additionally coupling or conjugating an anticancer drug to the cancer-homing-CPP the drug can be transported into breast cancer cells. This targeting can efficient the therapy while reducing side effects. When a diagnostic agent is coupled to the cancer-homing-CPP, the construct can be used for diagnostic purposes for detection of breast cancer cells by use of an appropriate method adapted for detection of the marker. When a diagnostic agent is coupled to a cancer-homing-CPP that carries an anticancer drug, the internalized construct can be used to monitor the effect of the anticancer drug on the breast cancer cells by use of an appropriate method adapted for detection of the marker.

One aspect of the invention is directed to a construct comprising a cancer-homing peptide, selected from a linear pentapeptide CREKA (SEQ ID NO:1), AREKA (SEQ ID NO: 23) or CREKA_{D} (SEQ ID NO: 23), and a cyclic nonapeptide CPGPEGAGC (SEQ ID NO:2), wherein the two cysteines are cydized, an optional linker and a cell-penetrating peptide coupled to an anticancer drug and/or a marker.

The optional linker can be an amino acid or peptide that does not interfere with the respective functions of the cancer-homing peptide and cell-penetrating peptide.

In another embodiment of the invention the cell-penetrating peptide is selected from the following peptide sequences:

| | | |
|---|---|---|
| Penetratin | RQIKIWFQNRRMKWKK | (SEQ ID NO:3) |
| Tat (48-60) | GRKKRRQRRRPPQ | (SEQ ID NO:4) |
| VP22 | DAATATRGRSAASRPTERPRAPARSASRPRRVD | (SEQ ID NO:5) |
| *p*VEC | LLIILRRRIRKQAHAHSK-amide | (SEQ ID NO:6) |
| *p*ISL | RVIRVWFQNKRCKDKK-amide | (SEQ ID NO:7) |
| hCT (9-32) derived peptide | LGTYTQDFNKFHTFPQTAIGVGAP | (SEQ ID NO:8) |
| LL-37 | LLGDFFRKSKEKIGKEFKRIVQRIKDFLRNLVPRTES | (SEQ ID NO:9) |
| Mouse PrP (1-28) | MANLGYWLLALFVTMWTDVGLCKKRPKP-amide | (SEQ ID NO:10) |
| Transportan (TP) | GWTLNSAGYLLGKINLKALAALAKKIL-amide | (SEQ ID NO:11) |
| TP10 | AGYLLGKINLKALAALAKKIL-amide | (SEQ ID NO:12) |
| Arg11 | RRRRRRRRRRR | (SEQ ID NO:13) |
| MAP | KLALKLALKALKAALKLA-amide | (SEQ ID NO:14) |
| Pep-1 | KETWWETWWTEWSQPKKKRKV | (SEQ ID NO:15) |
| Pep-2 | KETWFETWFTEWSQPKKKRKV | (SEQ ID NO:16) |
| MPG | GALFLGWLGAAGSTMGAPKKKRKV | (SEQ ID NO:17) |
| KALA | WEAKLAKALAKALAKHLAKALAKALKACEA | (SEQ ID NO:18) |
| ppTG1 | GLFKALLKLLKSLWKLLLKA | (SEQ ID NO:19) |
| ppTG20 | GLFRALLRLLRSLWRLLLRA | (SEQ ID NO:20) |

wherein the peptides are C-terminal free acids unless stated otherwise.

In a further embodiment of the invention, the anticancer drug is selected from alkylating agents, such as 4-[4-Bis(2-chloroethyl)amino)phenyl]butyric acid (Chlorambucil, further referred to as Cbl) or 3-[4-(Bis(2-chloroethyl)amino)phenyl]-L-alanine (Melphalan), antimetabolites, such as *N*-[4-(*N*-(2,4-Diamino-6-pteridinylmethyl)methylamino)-benzoyl]-L-glutamic acid (Methotrexate) and cytotoxic antibiotics, such as (8*S*,10*S*)-10-[(3-Amino-2,3,6-trideoxy-α-L-*ly*xo-hexopyranosyl)oxy]-8-glycoloyl-7,8,9,10-tetrahydro-6,8,11-trihydroxy-1-methoxy-5,12-naphthacenedione (Doxorubicin).

In yet another embodiment of the invention the diagnostic agent is a fluorescent label, such as a fluoresceinyl label.

In a preferred embodiment of the invention, the construct contains the peptide CPGPEGAGC-LLIILRRRIRKQAHAHSK-amide (SEQ ID NO:21).

In another preferred embodiment of the invention the construct contains the peptide CREKA-LLIILRRRIRKQAHAHSK-amide (SEQ ID NO:22).

Another aspect of the invention is directed to a conjugate for delivering an anticancer drug and/ or a diagnostic agent into a cancer cell comprising administration of a construct according to the invention in *vivo* to a subject expected to have breast cancer cells, such as breast cancer cells, or *in vitro* to a tissue sample or cell culture.

The invention will now be illustrated with reference to the drawings and the experiments.

### Description of the drawings

**Fig. 1****.** Scheme of chlorambucil-peptide conjugate. Chlorambucil is coupled to the N-terminus of the following peptides: *p*VEC (SEQ ID NO:6), CREKA (SEQ ID NO:1), CPGPEGAGC (SEQ ID NO:2), CREKA-*p*VEC (SEQ ID NO:22) and CPGPEGAGC-*p*VEC (SEQ ID NO:21).
**Fig. 2****.** Quantitative uptake of the peptides in MCF-7 and MDA-MB-231 cell lines. Cells were exposed to fluoresceinyl labeled *p*VEC (SEQ ID NO:6), CREKA-*p*VEC (SEQ ID NO:22) and CREKA (SEQ ID NO:1) solution (500 µl, 5 µM) for 30 min at 37 °C where after the cells were lysed and the amount of fluorescence in the cell lysate was measured and normalized to the protein content.
**Fig. 3****.** Quantitative uptake of the fluoresceinyl labeled peptides in MCF-7 and MDA-MB-231 cell lines. Cells were exposed to fluoresceinyl labeled CPGPEGAGC (PEGA, SEQ ID NO:2), CPGPEGAGC-*p*VEC (PEGA-pVEC, SEQ ID NO:21) and *p*VEC (SEQ ID NO:6) solution (500 µl, 5 µM) for 30 min at 37 °C where after the cells were lysed and the amount of fluorescence was measured and normalized to the protein content.
**Fig. 4****.** Microscopy uptake of the fluoresceinyl labeled CREKA-*p*VEC (SEQ ID NO:22) (A), *p*VEC (SEQ ID NO:6) (B) and CREKA (SEQ ID NO:1) (C) peptides in MDA-MB-435 cell line. Cells were exposed to a drug solution for 1 hour at 37 °C.
**Fig. 5****.** Microscopy uptake of the fluoresceinyl labeled CREKA-*p*VEC (SEQ ID NO:22) (A, 10 µM and B, 20 µM), *p*VEC (SEQ ID NO:6) (C, 20 µM) and CREKA (SEQ ID NO:1) (D, 20 µM) peptides in MDA-MB-435 cell line. Cells were exposed to a drug solution for 4 hours at 37 °C. The arrows indicate to the localization of CREKA-*p*VEC (SEQ ID NO:22) in the cell nucleus.
**Fig. 6****.** Microscopy uptake of the fluoresceinyl labeled peptides in MCF-7 cell line (bright field and fluorescence). Cells were exposed to a 5 µM drug solution for 45 min at 37 °C. *p*VEC (SEQ ID NO:6) (A), CREKA (SEQ ID NO:1) (B), CREKA-*p*VEC (SEQ ID NO:22) (C).
**Fig. 7****.** Microscopy uptake of the fluoresceinyl labeled peptides in MDA-MB-231 cell line (bright field and fluorescence). Cells were exposed to a 5 µM drug solution for 45 min at 37 °C. *p*VEC (SEQ ID NO:6) (A), CREKA (SEQ ID NO:1) (B), CREKA-*p*VEC (SEQ ID NO:22) (C).
**Fig. 8****.** Microscopy uptake of the fluoresceinyl labeled PEGA-*p*VEC (SEQ ID NO:21) (A), PEGA (SEQ ID NO:2) (B) and *p*VEC (SEQ ID NO:6) (C) peptides in MDA-MB-435 cell line. Cells were exposed to a drug solution for 1 hour at 37 °C.
**Fig. 9****.** Microscopy uptake of the fluoresceinyl labeled peptides in MCF-7 cell line (bright field and fluorescence). Cells were exposed to a 5 µM drug solution for 45 min at 37 °C, *p*VEC (SEQ ID NO:6) (A, B), PEGA (SEQ ID NO:2) (C, D) and PEGA-*p*VEC (SEQ ID NO:21) (E, F).
**Fig. 10****.** Microscopy uptake of the fluoresceinyl labeled peptides in MDA-MB-231 cell line (bright field and fluorescence). Cells were exposed to a 5 µM drug solution for 45 min at 37 °C. *p*VEC (SEQ ID NO:6) (A, B), PEGA (SEQ ID NO:2) (C, D) and PEGA-*p*VEC (SEQ ID NO:21) (E, F).
**Fig. 11****.** Cytotoxicity study of *p*VEC (SEQ ID NO:6) in MCF-7 cell line. Cells were exposed to different concentrations of *p*VEC solution in 150 µl of 1% FBS containing media and incubated for 2 h or 48 h at 37 °C. The plate was cooled to room temperature, CellTiter-Glo® Reagent (150 µl) was added and the luminescence was measured. The percent of viable cells was compared to the non-treated cells. These results show that *p*VEC (SEQ ID NO:6) itself at the concentrations used in this study is not toxic to the cells.

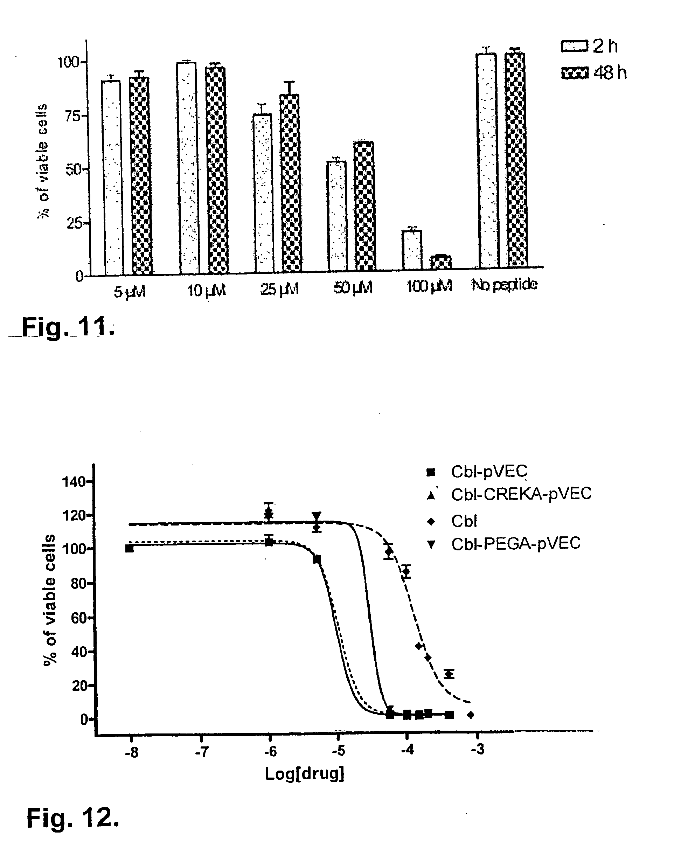

**Fig. 12****.** Cytotoxicity study of Cbl and Cbl conjugated peptides in MCF-7 cell line. Cells were exposed to different concentrations of Cbl, Cbl-*p*VEC (SEQ ID NO:6), Cbl-CREKA-*p*VEC (SEQ ID NO:22) and Cbl-PEGA-*p*VEC (SEQ ID NO:21) (0, 1, 5, 55, 100, 150, 200, 400 and 800 (only with Cbl) µM) solution in 150 µl of 1% FBS containing complete media and incubated for 3 h at 37 °C. After that 150 µl of 10% FBS containing media was added and the cells were incubated additionally for 45 h at 37 °C (altogether 48 h). The plate was cooled to room temperature, CellTiter-Glo® Reagent (150 µl) was added and the luminescence was measured. The percent of viable cells was compared to the non-treated cells. IC50 values were calculated using GraphPad Prism 4 software.

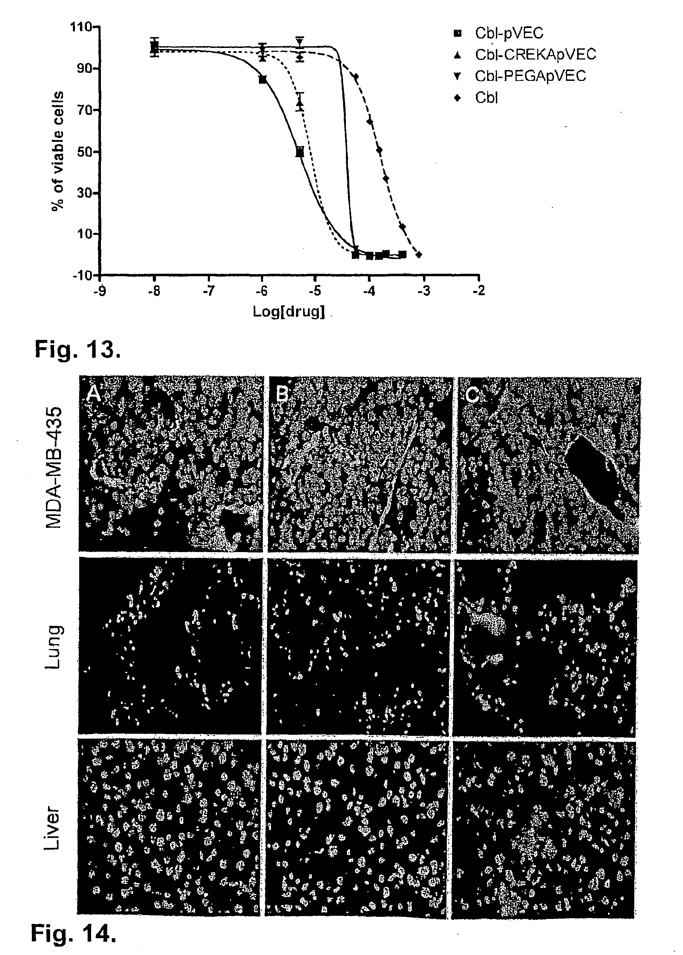

**Fig. 13****.** Cytotoxicity study of Cbl and Cbl conjugated peptides in MDA-MB-231 cell line. Cells were exposed to different concentrations of Cbl, Cbl-*p*VEC (SEQ ID NO:6), Cbl-CREKA-*p*VEC (SEQ ID NO:22) and Cbl-PEGA-*p*VEC (SEQ ID NO:21) (0, 1, 5, 55, 100, 150, 200, 400 and 800 (only with Cbl) µM) solution in 150 µl of 1% FBS containing complete media and incubated for 3 h at 37 °C. After that 150 µl of 10% FBS containing media was added and the cells were incubated additionally for 45 h at 37 °C (altogether 48 h). The plate was cooled to room temperature, CellTiter-Glo® Reagent (150 µl) was added and the luminescence was measured. The percent of viable cells was compared to the non-treated cells. IC50 values were calculated using GraphPad Prism 4 software.
**Fig. 14****.** Distribution of fluoresceinyl labeled CREKA-*p*VEC (SEQ ID NO:22) (A), CREKA (SEQ ID NO:1) (B) and *p*VEC (SEQ ID NO:6) (C) peptides in *vivo* 2 hours after the injection (MDA-MB-435 tumor, lung, liver).
**Fig. 15****.** Distribution of fluoresceinyl labeled CREKA-*p*VEC (SEQ ID NO:22) (A), CREKA (SEQ ID NO:1) (B) and *p*VEC (SEQ ID NO:6) (C) peptides *in vivo* 2 hours after the injection (spleen, heart, skin).
**Fig. 16****.** Distribution of fluoresceinyl labeled CREKA-*p*VEC (SEQ ID NO:22) (A), CREKA (SEQ ID NO:1) (B) and *p*VEC (SEQ ID NO:6) (C) peptides *in vivo* 2 hours after the injection (kidney, gut, brain).
**Fig. 17****.** Distribution of fluoresceinyl labeled CREKA (SEQ ID NO:1) (A), *p*VEC (SEQ ID NO:6) (B) and CREKA-*p*VEC (SEQ ID NO:22) (C) peptides *in vivo* 2 hours after the injection in MDA-MB-435 tumors. Confocal microscopy, 600 x magnifications. The arrows indicate the internalization of peptides into the cells in the tumor tissues.
**Fig. 18****.** Distribution of fluoresceinyl labeled CREKA-*p*VEC (SEQ ID NO:22) (A), CREKA (SEQ ID NO:1) (B) and *p*VEC (SEQ ID NO:6) (C) peptides *in vivo* 2 hours after the injection together with blood vessel marker (anti-MECA32, red fluorescence).
**Fig. 19****.** Distribution of fluoresceinyl labeled PEGA (SEQ ID NO:2) (A, B) and PEGA-*p*VEC (SEQ ID NO:21) (C, D, E, F) peptides *in vivo* 2 hours after the injection in MDA-MB-435 tumors. Confocal microscopy, 200 x magnifications (A-D) and 400 x magnifications (E, F).
**Fig. 20****.** Distribution of fluoresceinyl labeled PEGA-*p*VEC (SEQ ID NO:21) (A), PEGA (SEQ ID NO:2) (B) and *p*VEC (SEQ ID NO:6) (C) peptides in MDA-MB-435 tumor *in vivo* 2 hours after the injection. Distribution of fluoresceinyl labeled PEGA-*p*VEC (D), PEGA (E) and *p*VEC (F) peptides together with blood vessel marker (anti-MECA32, red fluorescence).
**Fig. 21****.** Distribution of fluoresceinyl labeled PEGA-*p*VEC (SEQ ID NO:21) (A), PEGA (SEQ ID NO:2) (B) and *p*VEC (SEQ ID NO:6) (C) peptides in lung, brain, heart and kidney *in vivo* 2 hours after the injection.
**Fig. 22****.** Distribution of fluoresceinyl labeled PEGA-*p*VEC (SEQ ID NO:21) (A), PEGA (SEQ ID NO:2) (B) and *p*VEC (SEQ ID NO:6) (C) peptides in liver, gut and skin *in vivo* 2 hours after the injection.
**Fig. 23****.** Distribution of fluoresceinyl labeled SAR CREKA (SEQ ID NO:1) (Ala1 (A)(SEQ ID NO: 23), Ala2 (B) (SEQ ID NO:24), Ala3 (C) (SEQ ID NO:25), Ala4 (D) (SEQ ID NO:26) and Ala5 (E) (SEQ ID NO:27))peptides in MDA-MB-435 tumor, lung and skin *in vivo* 24 hours after the injection.
**Fig. 24****.** Distribution of fluoresceinyl labeled SAR CREKA (SEQ ID NO:1) (Ala1 (A)(SEQ ID NO: 23), Ala2 (B) (SEQ ID NO:24), Ala3 (C) (SEQ ID NO:25), Ala4 (D) (SEQ ID NO:26) and Ala5 (E) (SEQ ID NO:27)) peptides in brain, heart, kidney *in vivo* 24 hours after the injection.
**Fig. 25****.** Distribution of fluoresceinyl labeled SAR CREKA (SEQ ID NO:1) (Ala1 (A)(SEQ ID NO: 23), Ala2 (B) (SEQ ID NO:24), Ala3 (C) (SEQ ID NO:25), Ala4 (D) (SEQ ID NO:26) and Ala5 (E) (SEQ ID NO:27))peptides in spleen, gut and liver *in vivo* 24 hours after the injection.
**Fig. 26****.** The inhibition of MDA-MB-435 tumor growth in mice with the Cbl-CREKA-*p*VEC (SEQ ID NO:22) conjugates. The MDA-MB-435 orthotopic xenografted mice (8 mice/group) were systemically treated with 15 µg of Cbl equivalent of Cbl-CREKA-*p*VEC (SEQ ID NO:22) and of the controls. A significant decrease in tumor growth was observed in tumors of mice treated with the Cbl-CREKA-*p*VEC (SEQ ID NO:22) conjugate compared with controls (P < 0.01).

### Experiments

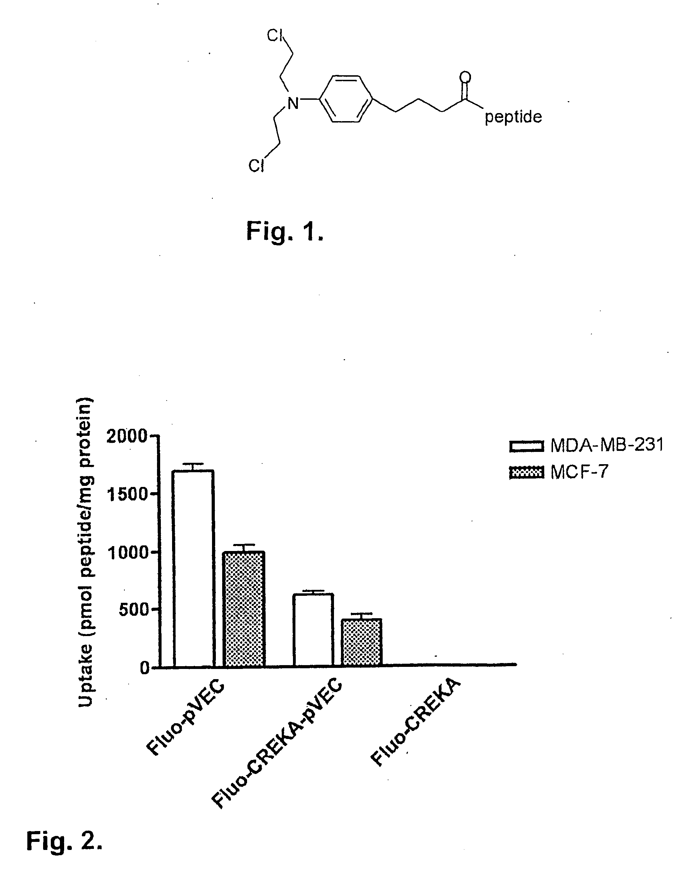

In this study, two cancer homing peptides, a cyclic nonapeptide CPGPEGAGC (SEQ ID NO:2) (cyclic between the two cysteines) ²⁵ and a linear pentapeptide CREKA (SEQ ID NO:1), have been coupled to the N-terminus of the cell-penetrating peptide *p*VEC ¹⁴ (SEQ ID NO:6) in order to achieve the synergistic properties of both components, i.e. the cancer cell targeting together with cellular penetration of the construct. A well-known anticancer drug, chlorambucil (Fig. 1) has been coupled to the N-terminus of the chimeric peptide in order to achieve the toxicity to cancer cells selectively.

### Materials and methods

### Peptide synthesis

Peptides (Table 1) were synthesized automatically (model 431 A; Applied Biosystems) on solid support p-methylbenzhydrylamin resin (Neosystem) (substitution 1.16 mmol/g) generating C-terminally amidated peptides according to manufactorers instructions. Stepwise coupling reactions were performed with *t*-Boc-protected amino acids (Neosystem), 1-hydroxybenzotriazole, N, N'-dicyclohexylcarbodiimide (4:4:4 eq, 35 min, RT) followed by N-terminal deprotection of the *t*-Boc-group with TFA: dichloromethane (1:1) (14 min, RT).

For cellular uptake and *in vivo* studies the peptides were labeled N-terminally with 5(6)-carboxyfluorescein (Molecular Probes) (3 eq) in N,N'-diisipropylcarbodiimide (3 eq) and hydroxybenzotriazole (3 eq) in dimethyl sulfoxide:dimethylformamide (1:2) in dark overnight.

For cytotoxicity studies chlorambucil (4 eq) (Sigma-Aldrich) was coupled to N-terminal amino group of the peptides with 2-(1H-benzotriazole-1-yl)-1,1,3,3-tetramethyluronium tetrafluoroborate (4 eq), 1-hydroxybenzotriazole (4 eq) and diisopropylethylamine (8 eq) in dimethylformamide for 30 min.

Deprotection of the dinitrophenyl group was performed by treating the peptides with thiophenol:dimethylformamide (1:4) (1h, RT). Final cleavage of the peptides from the resin was performed in hydrofluoric acid (1 h, 0 °C) in the presence of *p*-cresol and p-thlocresol.

Fluoresceinyl labeled or chlorambucil coupled CPGPEGAGC-amide (SEQ ID NO:2) (in text and figures referred to as PEGA) and CPGPEGAGC-*p*VEC-amide (SEQ ID NO:21) (in text and figures referred to as PEGA-*p*VEC) were incubated overnight in dimethyl sulfoxide generating disulfide bridges between cysteines.

Peptides (Table 3) were synthesized automatically (Syro2000 Multiple Peptide Synthesizer; MultiSynTech GmbH) on solid support RINK amide methylbenzhydrylamin resin (Neosystem) (substitution 0.85 mmol/g) generating C-terminally amidated peptides according to manufactorers instructions. Stepwise coupling reactions were performed with Fmoc-protected amino acids (MultiSynTech GmbH), 2-(1H-benzotriazole-1-yl)-1,1,3,3-tetramethyluronium tetrafluoroborate, diisopropylethylamine (4:8:8 eq, 30 min, RT) followed by N-terminal deprotection of the Fmoc-group with 20% piperidine in DMF (v/v) (20 min, RT).

For *in vivo* studies the peptides were labeled N-terminally with 5(6)-carboxyfluorescein (Molecular Probes) (3 eq) in N,N'-diisipropylcarbodiimide (3 eq) and hydroxybenzotriazole (3 eq) in dimethyl sulfoxide:dimethylformamide (1:2) in dark overnight.

Final cleavage of the peptides from the resin was performed using TFA-scavengers cocktail. Standard reagent K content was modified to prevent irreversible modification of product by linker. The cocktail mixture contained 95% TFA, 2.5% water, 2.5% TIS (sometimes with addition of 2.5% thioanisole).

The peptides (Table 1 and Table 3) were purified by RP-HPLC (Discovery C-18 HPLC column, 25 cm, 21.2 mm, 5 µm) using a gradient of acetonitrile/water with 0.1% TFA (50 min, 2 ml/min). The identity and quality of the purified products were verified by matrix assisted laser desorption ionization time-of-flight mass-spectrometer (prOTOF^{™} 2000 MALDI O-TOF, PerkinElmerSCIEX). The mass-spectra were acquired in positive ion reflector mode using α-cyano-4-hydroxycinnamic acid as a matrix (Sigma-Aldrich) (10 mg/ml, 7:3 acetonitrile:water, 0.1 % TFA).

### Cell culture

The human breast cancer cell line MDA-MB-231 and the human breast cancer cell line MCF-7 (ATCC via LGC, Sweden) were cultured in RPMI-1640 supplemented with 10% fetal bovine serum, sodium pyruvate (1 mM), penicillin (100 U/ml), streptomycin (100 µg/ml) and 1% nonessential amino acids. The human breast cancer cell line MDA-MB-435 was maintained in DMEM supplemented with 10% FCS, penicillin (100 U/ml), and streptomycin (100 µg/ml). All cells were incubated at 37 °C in 5% CO₂.

### Quantitative uptake for CREKA and CPGPEGAGC peptides

MCF-7 and MDA-MB-231 cells (150 000 cells/well) were seeded on a 12 well plate two days before the experiment. The cells were washed with HEPES buffered krebs ringer solution (HKR) (2x1 ml) and exposed to a drug solution (500 µl, 5µM) for 30 min at 37 °C. The cells were washed with HKR (2x1 ml) and treated with trypsin-EDTA (200 µl) for 5 min at 37 °C. HKR (1 ml) was added and the cells were transferred to eppendorf tubes and centrifuged (1000xg) for 5 min at 4 °C. Cell pellet was lysed with NaOH (300 µl, 0,1 M) for 1 h at 4 °C and centrifuged (10000xg) for 10 min at 4 °C. The fluorescence (494/518 nm) was measured with SPEKTRAmax® GEMINI XS spectrofluorometric plate reader (Molecular Devices).

### Microscopy study for peptide uptake

MCF-7 and MDA-MB-231 cells (20 000 cells/well) were seeded two days before the experiment on a Lab-Tek® 8-well chamber slides. The cells were washed with HKR (2x400 µl) and exposed to drug solution (400 µl, 5 µM) for 45 min at 37 °C. The cells were washed with HKR (3x400 µl) where after the pictures were taken with UltraView ERS Confocal Live Cell Imager microscope (Zeiss, PerkinElmer).

MDA-MB-435 cells (10 000 cells/well) were seeded one day before the experiment on a Lab-Tek® 8-well chamber slides. The cells were washed with serum-free DMEM (2x300 µl) and exposed to drug solution (300 µl, 10 µM or 20µM) for 1h or 4h at 37 °C. The cells were washed five times (5x300 µl) with PBS, fixed with 4% PFA at room temperature for 15 minutes and washed two times with PBS. The slides were mounted in Vectashield Mounting Medium with DAPI (Vector Laboratories, Burlingame), and observed under a Radiance MP confocal microscope.

### Toxicity study

MCF-7 and MDA-MB-231 cells (20 000 cells/well) were seeded in a 48 well plate two days before the experiment. The cells were exposed to drug in different concentration in full media containing 1% FBS for 3 h where after 10% FBS containing full media was added to achieve 5% FBS containing media and then incubated 45 or 69 h at 37 °C. The plate was cooled to room temperature and CellTiter-Glo® Reagent (Promega) was added. The plate was incubated for 10 min at room temperature where after the luminescence was measured.

### In vivo homing analysis of chimeric peptides

To produce tumors, nude BALB/c mice were orthotopically injected with 1X10⁶ MDA-MB-435 tumor cells into mammary fat pad. The tumor bearing mice were used for homing analysis of peptides when the tumor size reached about 10 mm. Tissue distribution of fluoresceinyl labeled peptides was studied by intravenously injecting the peptides (100 µM in 200 µl PBS) into tumor-bearing mice. The injected peptides were allowed to circulate 2 or 24 h, and the mice were perfused with 4% paraformaldehyde through the left ventricle of heart. Tissues were dissected and frozen in OCT embedding medium (Tissue-Tek, Elkhart, IN). Frozen sections were prepared for immunohistological analysis.

### Treatment of mice bearing MDA-MB-435 tumors with Cbl-CREKA-pVEC (SEQ ID NO:1) peptide conjugates

The orthotopic xenografted breast tumors were established by injecting 1 X 10⁶ MDA-MB-435 human breast cancer cells into the mammary fat pad of nude Balb/c mice. Treatment started when mean tumor volumes reached about 100 mm³. Mice with size-matched tumors were randomized into five groups (eight animals per group): Cbl-CREKA-*p*VEC (SEQ ID NO:22), Cbl plus CREKA-*p*VEC, Cbl-CREKA (SEQ ID NO:1), Cbl-*p*VEC (SEQ ID NO:6) and PBS. Mice were treated intravenously with 15 µg of Cbl-equivalent/mouse every other day for seven times. Tumor size was measured every three days. The mice were monitored for weight loss. The animal experiments reported here were approved by the Animal Research Committee of Burnham Institute for Medical Research.

### Results

### In vitro studies

### Uptake studies

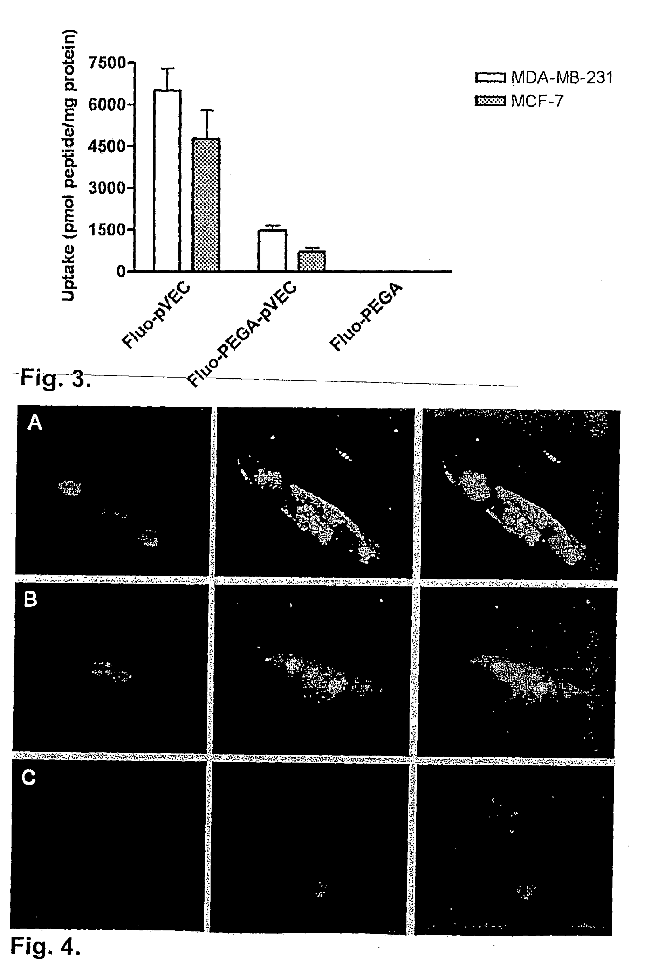

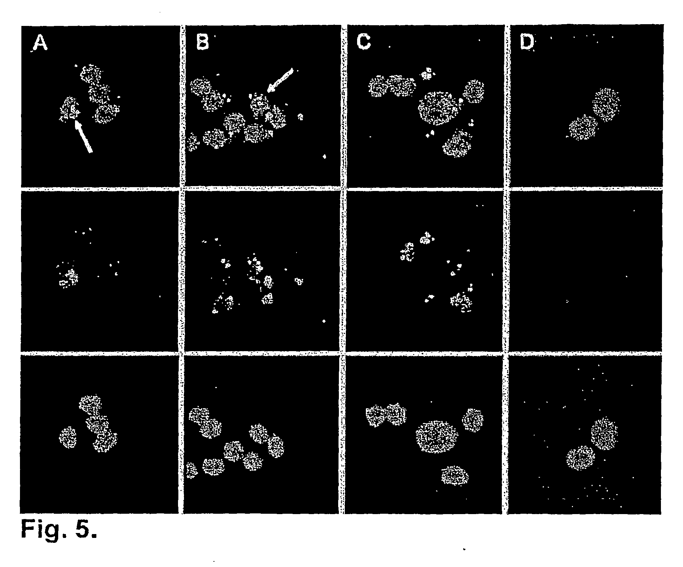

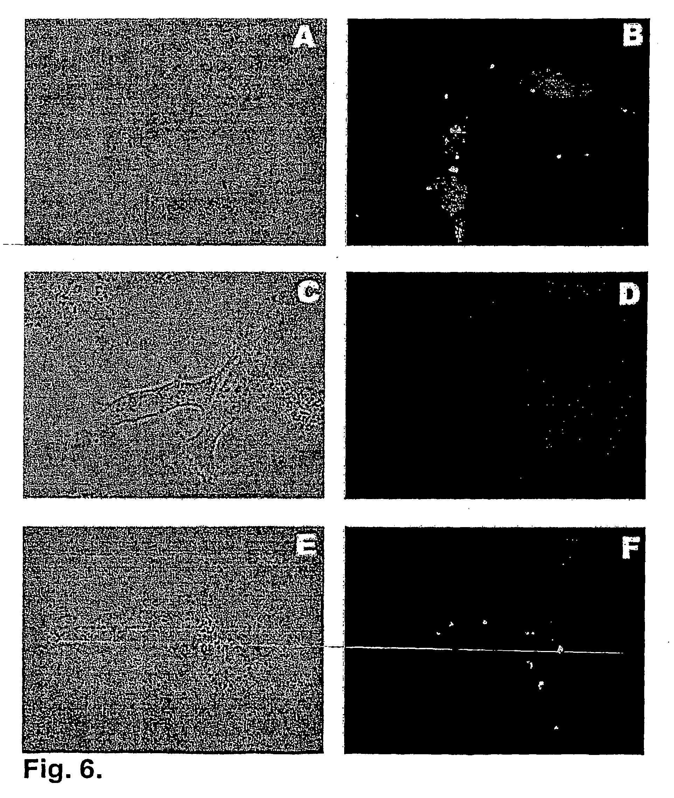

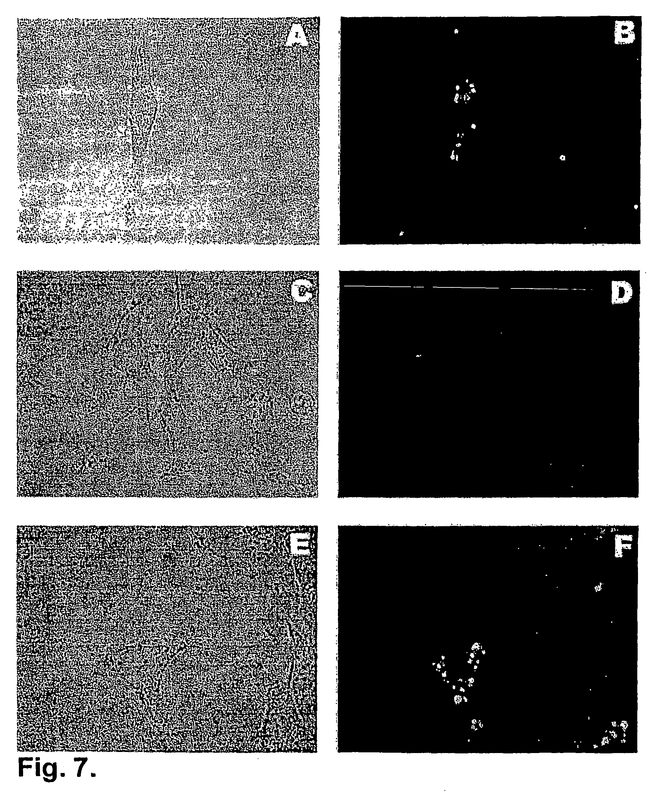

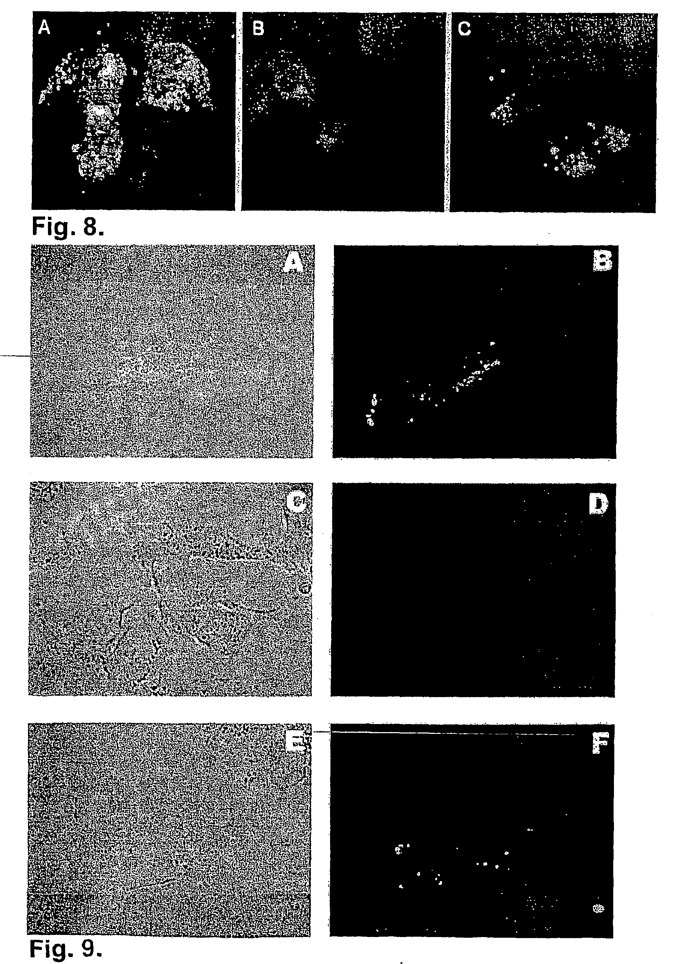

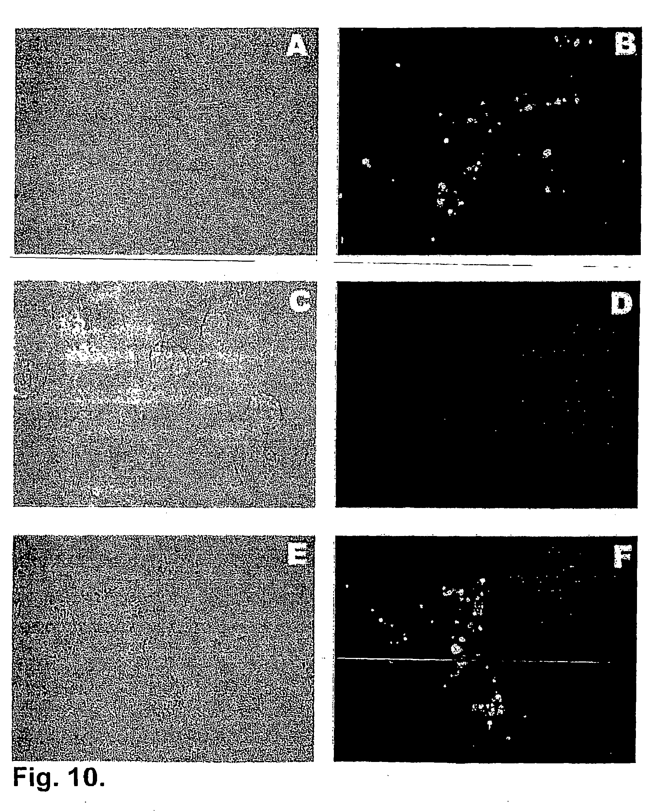

To determine the cell-penetrating properties of the homing peptides alone and homing-CPP chimeras the uptake of the fluoresceinyl labeled peptides was investigated quantitatively (Fig. 2 and 3) and by microscopy (Fig. 4, 5, 6, 7, 8, 9 and 10). We confirmed that the homing peptides used in this study did not alone display cell-penetrating properties, but when conjugated to *p*VEC (SEQ ID NO:6) entered into cultured MDA-MB-231, MDA-MB-435 and MCF-7 cells. The CREKA-*p*VEC (SEQ ID NO:22) peptide appears in the nucleus of MDA-MB-435 cells after 4h incubation at 37°C (Fig. 5).

### Toxicity studies of chlorambucil and chlorambucil conjugates

To determine that the concentration (10 µM) used for *p*VEC (SEQ ID NO:6) and its conjugates for uptake studies are not toxic, the toxicity of different concentrations of *p*VEC was measured (Fig. 11).

Coupling a cytotoxic agent to the homing conjugate could direct the drug into tumors. Chlorambucil is a cytotoxic agent that acts inside the cell. It alkylates DNA and prevents proliferation of cancer cells and may also induce apoptosis. The ability of the chimeric peptides to internalize chlorambucil as measured by the ability of the conjugates to kill tumor cells was investigated in two different cell lines, MCF-7 and MDA-MB-231. Cbl-CREKA-*p*VEC (SEQ ID NO:22) improved Cbl toxicity both in MCF-7 (IC50 value 10.5 µM) (Fig. 12) and MDA-MB-231 (IC50 value 8.2 µM) (Fig. 13) cells as compared to Cbl alone (IC50 values 128.4 µM and 164.0 µM, respectively). Cbl-PEGA-*p*VEC (SEQ ID NO:21) improved also Cbl toxicity both in MCF-7 (IC50 value 29.9 µM) (Fig. 12) and MDA-MB-231 (IC50 value 37.4 µM) (Fig. 13) as compared to Cbl alone (IC50 values 128.4 µM and 164.0 µM, respectively).

### In vivo studies

### Homing specificity of chimeric pVEC (SEQ ID NO:6) peptides

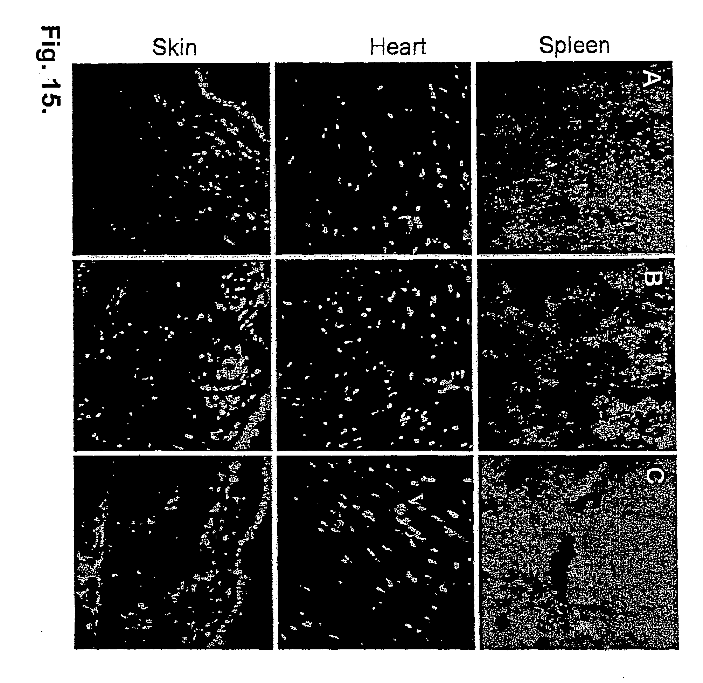

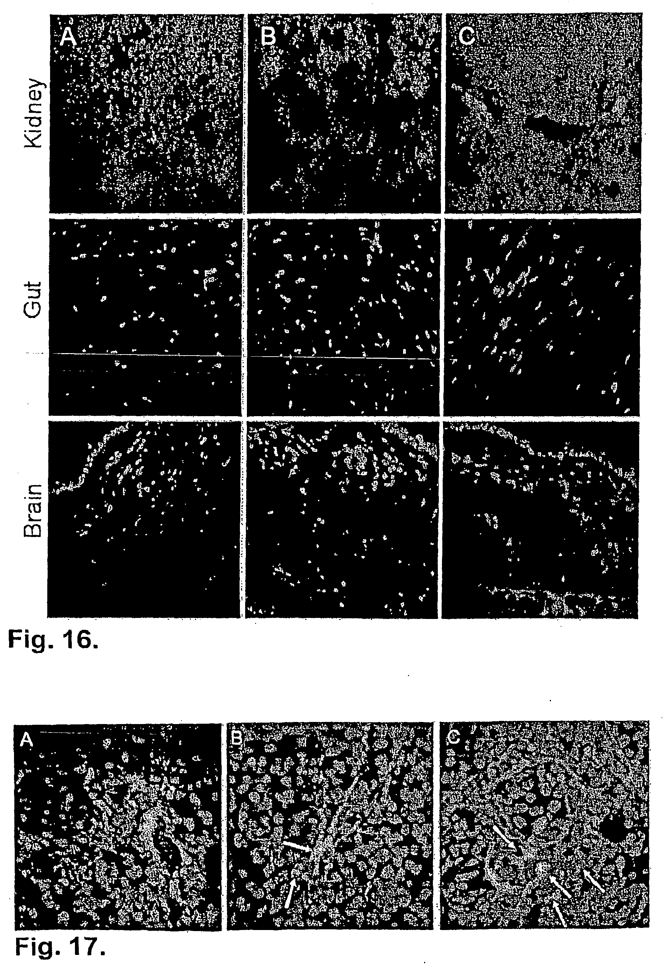

To evaluate the homing specificity of the CREKA-*p*VEC (SEQ ID NO:22), the mice bearing MDA-MB-435 tumors were intravenously injected with 100 µM of fluoresceinyl labeled CREKA-*p*VEC chimeric peptide (SEQ ID NO:22) or an equimolar amount of fluoresceinyl labeled CREKA (SEQ ID NO:1) or fluoresceinyl labeled *p*VEC (SEQ ID NO:6) as controls. The results showed that the chimeric peptide strongly homes to tumors, but not to control organs such as liver, heart, skin, kidney, gut and brain. The pattern of tissue distribution of this chimeric peptide is similar to that of CREKA peptide (SEQ ID NO:1) alone except the chimeric peptide is weakly positive in lung and spleen (Fig. 14, 15, 16, 17). In contrast, the non-targeted, fluoresceinyl labeled *p*VEC peptide (SEQ ID NO:6) accumulated in all tissues (Fig. 14, 15, 16, 17). CREKA-*p*VEC (SEQ ID NO:22) appears in the nucleus of cells in tumors (Fig.17 C). These data show that each of the two peptides endow the chimera with its main desired property, specific homing to tumors and internalization into the cells at the target.

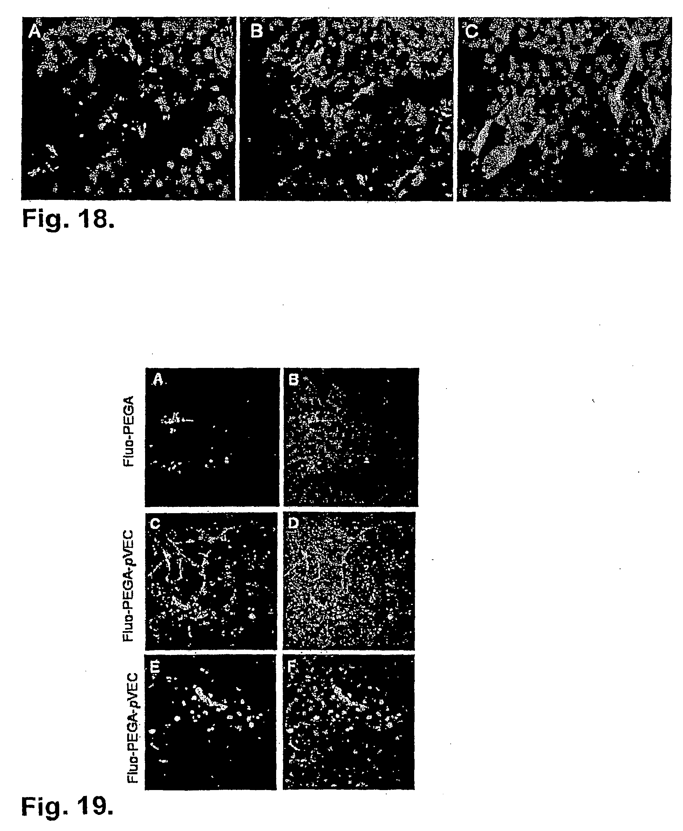

To study the association of CREKA-*p*VEC peptides (SEQ ID NO:22) with the vasculature, fluoresceinyl labeled chimeric or control peptides were intravenously injected into MDA-MB-435 tumor bearing mice, and peptide localization was compared to blood vessel markers localized with anti-MECA32 antibody. The chimeric peptide showed substantial co-localization with the MECA-32 (Fig.18). This data suggested that CREKA-*p*VEC (SEQ ID NO:22) recognizes tumor blood vessels.

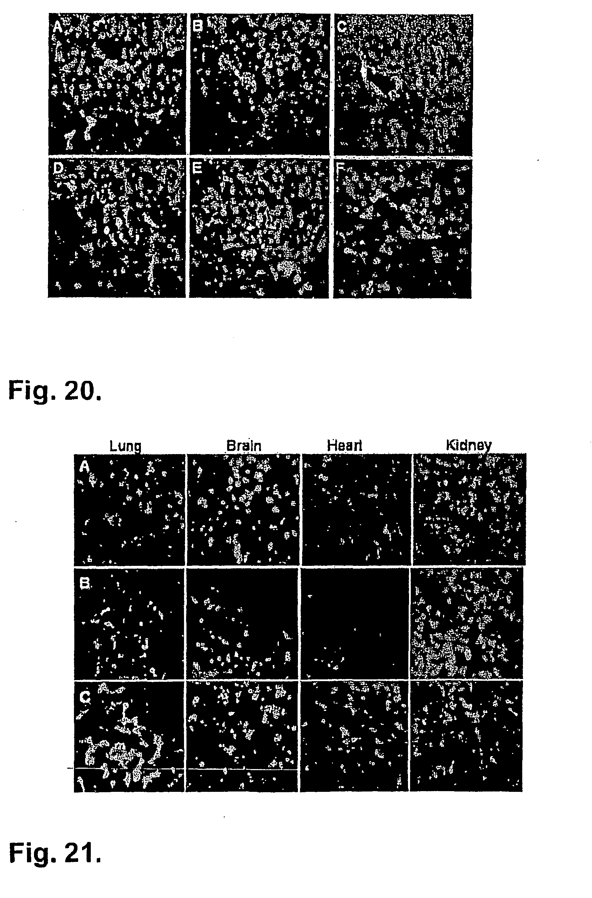

A chimeric peptide composed of *p*VEC (SEQ ID NO:6) coupled to another tumor-homing peptide, CPGPEGAGC ²⁵(SEQ ID NO:2) was also studied. This chimeric peptide, PEGA-*p*VEC (SEQ ID NO:21), showed less clear increase of tumor specificity than the CREKA (SEQ ID NO:22) conjugate, but similar to was observed with the CREKA (SEQ ID NO:22) conjugates, the homing peptide appeared to reduce the accumulation of the peptide in non-tumor tissues (Fig. 19, 20, 21 and 22).

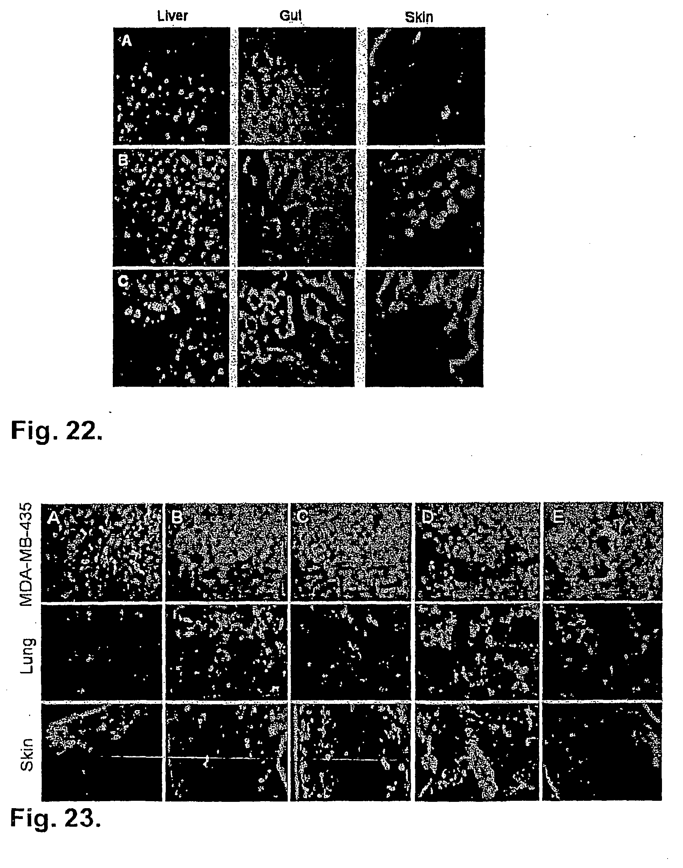

The tissue distribution and localization of CPGPEGAGC-*p*VEC (referred as PEGA-*p*VEC) (SEQ ID NO:21) were analyzed by using the same method as described above Fig. 19, 20, 21 and 22). As shown in Fig.23, the PEGA-*p*VEC chimeric peptide (SEQ ID NO:21) has a similar pattern of tissue distribution to that of *p*VEC (SEQ ID NO:6) among the tumor and various control organs, although the accumulation of PEGA-*p*VEC (SEQ ID NO:21) was observed at a lesser extent among some control organs such as liver, spleen and brain.

### Homing specificity of SAR CREKA peptides (SEQ ID NO:1)

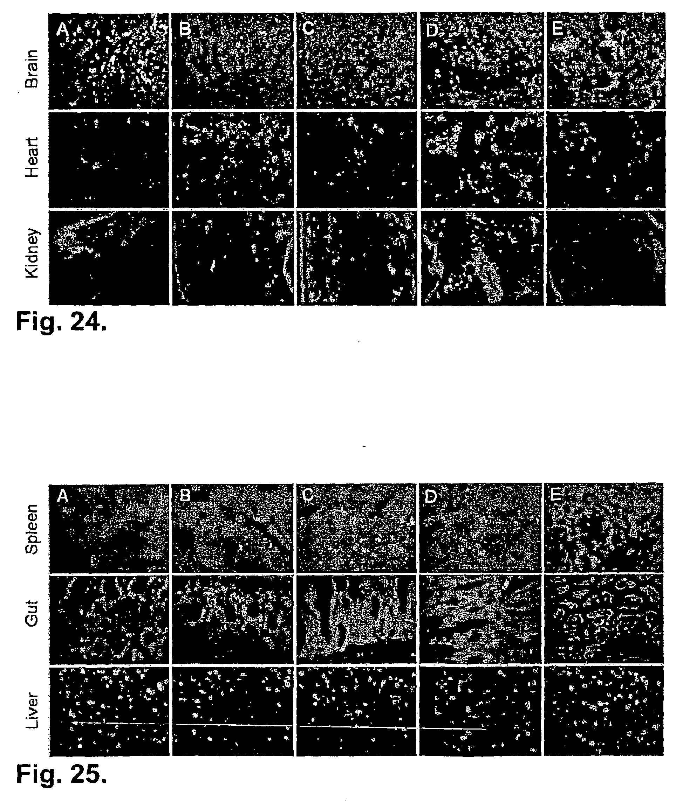

A partial structure-activity analysis (SAR) was performed with the CREKA peptide. One amino acid at a time was replaced with alanine, except that the alanine in the original peptide was converted to a D-alanine (Table 3). Fluoresceinyl labeled peptides (100 µM) were intravenously injected into the mice bearing MDA-MB-435 tumors. After 24 h circulation, tumors and various control organs were dissected for histology analysis. The results show that the REK motif is critical for CREKA peptide *(SEQ ID NO:1)* homing to MDA-MB-435 tumors (Fig. 23, 24, 25, Table 4). The peptides AREKA (SEQ ID NO: 23) and CREKA_{D} (SEQ ID NO: 27) have the same the distribution in different tissues *in vivo* as the original CREKA peptide (SEQ ID NO:1).

### Treatment of mice bearing MDA-MB-435 tumors with cbl-CREKA-pVEC peptide conjugates (SEQ ID NO:22)

To determine whether the CREKA-*p*VEC peptides (SEQ ID NO:22) could be used to improve the therapeutic index of cancer chemotherapeutics, we coupled them with chlorambucil. The Cbl-CREKA-*p*VEC conjugates were used to treat mice bearing MDA-MB-435 xenograft tumors.

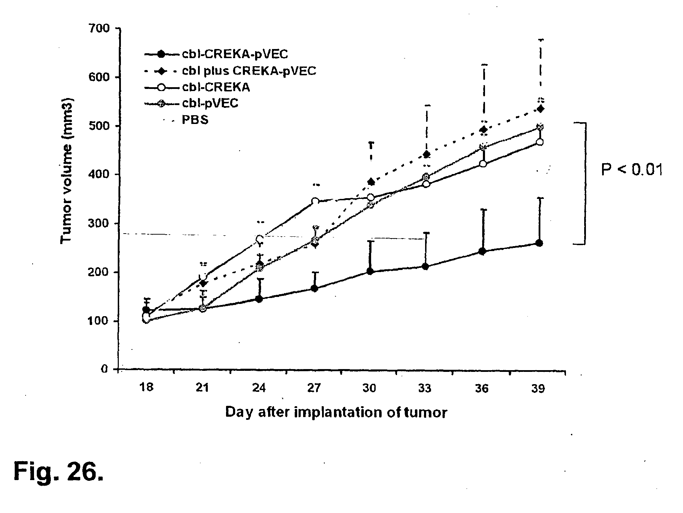

The previous report showed that the ED_{15d} dose of Cbl in tumor mice is 15 mg/Kg (about 300 µg per mouse for 15 days) ²⁹. Because we expected the Cbl conjugates to be more effective than the free drug, the tumor mice were treated with drug conjugates at a dose of Cbl-equivalent of 15 µg per mouse every other day for 14 days and were then observe. As shown in Fig 26, treatment with Cbl-CREKA-*p*VEC (SEQ ID NO:22) conjugates resulted in a significant inhibition of tumor growth compared with control groups (p < 0.01). The data suggested that the therapeutic efficacy of free Cbl to MDA-MB-435 tumor was markedly enhanced when it conjugated to CREKA-*p*VEC peptides (SEQ ID NO:22). No significant differences were observed in the weight of the mice belonging to the various treatment groups, indicating lack of general overt toxicity.

### Summary of results

The cyclic peptide CPGPEGAGC-amide (SEQ ID NO:2) and the linear peptides CREKA-amide (SEQ ID NO:1), AREKA-amide (SEQ ID NO: 23) and CREKA_{D}-amide (SEQ ID NO: 27) are not cell penetrating by themselves. By conjugating the peptides to the cell penetrating peptide *p*VEC, LLIILRRRIRKQAHAHSK-amide (SEQ ID NO:6), they can be transported inside the cell *in vitro* and *in vivo*. The conjugates CPGPEGAGCLLIILRRRIRKQAHAHSK-amide (SEQ ID NO:21) and CREKA-LLIILRRRIRKQAHAHSK-amide (SEQ ID NO:22) internalize into three different cell lines MCF-7, MDA-MB-231 and MDA-MB-435. The homing specificity of CREKA-*p*VEC (SEQ ID NO:22) to tumor is better than that of PEGA-*p*VEC (SEQ ID NO:21) (Table 2). The REK motif is critical for CREKA (SEQ ID NO:1) homing to tumor *in vivo*. The treatment of mice bearing MDA-MB-435 tumor with Cbl-CREKA-*p*VEC (SEQ ID NO:22) conjugates resulted in a significant inhibition of tumor growth compared with control groups (p < 0.01).

It should be understood that the invention is not limited to the specifically disclosed examples and that modifications there are within the scope of this invention. The teachings of the cited literature are incorporated herein by reference.

### References

1. Derossi, D., Joliot, A. H., Chassaing, G. & Prochiantz, A. (1994). The third helix of the Antennapedia homeodomain translocates through biological membranes. J Biol Chem 269, 10444-50.
2. Eiriksdottir, E., Myrberg, H., Hansen, M. & Langel, Ü. (2004). Cellular uptake of cell-penetrating peptides. Drug Design Reviews 1, 161-173.
3. Stein, S., Weiss, A., Adermann, K., Lazarovici, P., Hochman, J. & Wellhoner, H. (1999). A disulfide conjugate between anti-tetanus antibodies and HIV (37-72)Tat neutralizes tetanus toxin inside chromaffin cells. FEBS Lett. 458, 383-6.
4. Pooga, M., Kut, C., Kihlmark, M., Hällbrink, M., Fernaeus, S., Raid, R., Land, T., Hallberg, E., Bartfai, T. & Langel, Ü. (2001). Cellular translocation of proteins by transportan. Faseb J. 15, 1451-3.
5. Pooga, M., Hällbrink, M., Zorko, M. & Langel, Ü. (1998). Cell penetration by transportan. Faseb J. 12, 67-77.
6. Rousselle, C., Clair, P., Lefauconnier, J. M., Kaczorek, M., Scherrmann, J. M. & Temsamani, J. (2000). New advances in the transport of doxorubicin through the blood-brain barrier by a peptide vector-mediated strategy. Mol. Pharmacol. 57, 679-86.
7. Mann, D. A. & Frankel, A. D. (1991). Endocytosis and targeting of exogenous HIV-1 Tat protein. Embo J 10, 1733-9.
8. Langel, Ü, (2002). Cell-penetrating peptides, processes and applications, Ed., CRC: Boca Raton, Press.
9. Fisher, L., Soomets, U., Cortes Toro, V., Chilton, L., Jiang, Y., Langel, Ü. & Iverfeldt, K. (2004). Cellular delivery of a double-stranded oligonucleotide NFkappaB decoy by hybridization to complementary PNA linked to a cell-penetrating peptide. Gene Ther. 11, 1264-72.
10. Fuchs, S. M. & Raines, R. T. (2004), Pathway for polyarginine entry into mammalian cells. Biochemistry 43, 2438-44.
11. Vivès, E., Richard, J. P., Rispal, C. & Lebleu, B. (2003). TAT peptide internalization: seeking the mechanism of entry. Curr. Protein. Pept. Sci. 4, 125-32.
12. Järver, P. & Langel, Ü. (2004). The use of cell-penetrating peptides as a tool for gene regulation. Drug Discov. Today 9, 395-402.
13. Joliot, A. & Prochiantz, A. (2004). Transduction peptides: from technology to physiology. Nat. Cell. Biol. 6, 189-96.
14. Elmquist, A., Lindgren, M., Bartfai, T. & Langel, Ü. (2001). VE-cadherin-derived cell-penetrating peptide, pVEC, with carrier functions. Exp. Cell. Res. 269, 237-44.
15. Elmquist, A. & Langel, Ü. (2003). In vitro uptake and stability study of pVEC and its all-D analog. Biol. Chem. 384, 387-93.
16. Conner, S. D. & Schmid, S. L. (2003). Regulated portals of entry into the cell. Nature 422, 37-44.
17. Mukherjee, S., Ghosh, R. N. & Maxfield, F. R. (1997). Endocytosis. Physiol. Rev. 77, 759-803.
18. Capecchi, M. R. (1980). High efficiency transformation by direct microinjection of DNA into cultured mammalian cells. Cell 22, 479-88.
19. Neumann, E., Schaefer-Ridder, M., Wang, Y. & Hofschneider, P. H. (1982). Gene transfer into mouse lyoma cells by electroporation in high electric fields. Embo J. 1, 841-5.
20. Ruoslahti, E. (2002). Drug targeting to specific vascular sites. Drug Discov Today 7, 1138-43.
21. Ruoslahti, E. (2002). Specialization of tumor vasculature. Nat Rev Cancer 2, 83-90.
22. Ruoslahti, E. (2004). Vascular zip codes in angiogenesis and metastasis. Biochem Soc Trans 32, 397-402.
23. Arap, W., Pasqualini, R. & Ruoslahti, E. (1998). Cancer treatment by targeted drug delivery to tumor vasculature in a mouse model. Science 279, 377-80.
24. Hoffman, J. A., Giraudo, E., Singh, M., Zhang, L., Inoue, M., Porkka, K., Hanahan, D. & Ruoslahti, E. (2003). Progressive vascular changes in a transgenic mouse model of squamous cell carcinoma. Cancer Cell 4, 383-91.
25. Essler, M. & Ruoslahti, E. (2002). Molecular specialization of breast vasculature: a breast-homing phage-displayed peptide binds to aminopeptidase P in breast vasculature. Proc Natl Acad Sci U S A 99, 2252-7.
26. Laakkonen, P., Porkka, K., Hoffman, J. A. & Ruoslahti, E. (2002). A tumor-homing peptide with a targeting specificity related to lymphatic vessels. Nat Med 8, 751-5.
27. Ellerby, H. M., Arap, W., Ellerby, L. M., Kain, R., Andrusiak, R., Rio, G. D., Krajewski, S., Lombardo, C. R., Rao, R., Ruoslahti, E., Bredesen, D. E. & Pasqualini, R. (1999). Anti-cancer activity of targeted pro-apoptotic peptides. Nat Med 5, 1032-8.
28. Chen, Y., Xu, X., Hong, S., Chen, J., Liu, N., Underhill, C. B., Creswell, K. & Zhang, L. (2001). RGD-Tachyplesin inhibits tumor growth. Cancer Res 61, 2434-8.
29. Lee, F.Y. & Workman, P. (1986). Altered pharmacokinetics in the mechanism of chemosensitization: effects of nitroimidazoles and other chemical modifiers on the pharmacokinetics, antitumour activity and acute toxicity of selected nitrogen mustards. Cancer Chemother Pharmacol 17(1), 30-7.

**Table 1. Sequences of peptides used in this study.**

| **Code no.** | **Peptide** | **Sequence** | **SEQ ID NO:** |
|---|---|---|---|
| M838 | pVEC | LLIILRRRIRKQAHAHSK-amide | SEQ ID NO:6 |
| G68 | CREKA | CREKA-amide | SEQ ID NO:1 |
| M914 | Cyclic PEGA | CPGPEGAGC-amide | SEQ ID NO:2 |
| M915 | CREKA-pVEC | CREKALLIILRRRIRKQAHAHSK-amide | SEQ ID NO:22 |
| M923 | Cyclic PEGA-pVEC | CPGPEGAGCLLIILRRRIRKQAHAHSK-amide | SEQ ID NO:21 |

**Table 2. Summary of distribution of fluoresceinyl labeled CREKA-pVEC (SEQ ID NO:22) and PEGA-pVEC (SEQ ID NO:21) in vivo.**

| | CREKA-*p*VEC | CREKA | *p*VEC | PEGA-*p*VEC | PEGA |
|---|---|---|---|---|---|
| Tumor | ++ | ++ | ++ | + | + |
| Lung | +/- | - | ++ | ++ | |
| Heart | - | - | - | - | - |
| Liver | - | - | ++ | + | + |
| Spleen | +/- | - | ++ | + | + |
| Gut | +/- | +/- | +/- | +/- | +/- |
| Brain | + | + | ++ | + | +/- |
| Skin | - | - | + | + | + |
| Kidney | +/- | - | +/- | + | +/- |

| | | | | | |
|---|---|---|---|---|---|
| ++, strong positive, +, positive, +/-, weak positive or negative, -, negative. | | | | | |

**Table 3. Substitution of amino acids with alanine in CREKA sequence (SEQ ID NO:1).**

| **Peptide** | **Sequence** | **SEQ ID NO:** |
|---|---|---|
| Ala1 | AREKA-amide | SEQ ID NO: 23 |
| Ala2 | CAEKA-amide | SEQ ID NO: 24 |
| Ala3 | CRAKA-amide | SEQ ID NO: 25 |
| Ala4 | CREAA-amide | SEQ ID NO: 26 |
| Ala5 | CREKA_{D}-amide* | SEQ ID NO: 27 |

| | | |
|---|---|---|
| * L-alanine is substituted with D-alanine | | |

**Table 4. Summary of distribution of fluoresceinyl labeled SAR CREKA peptides in vivo and comparison with the distribution of the original CREKA peptide(SEQ ID NO:1).**

| | Ala1 | Ala2 | Ala3 | Ala4 | Ala5 | CREKA |
|---|---|---|---|---|---|---|
| Tumor | ++ | - | +/- | +/- | ++ | ++ |
| Lung | - | ++ | - | ++ | - | - |
| Skin | - | ++ | +/- | + | - | - |
| Brain | +/- | +/- | + | + | +/- | +/- |
| Heart | - | - | - | - | - | - |
| Kidney | - | - | - | - | - | - |
| Spleen | - | + | + | + | +/- | - |
| Gut | +/- | + | + | + | +/- | +/- |
| Liver | - | - | - | - | - | - |

| | | | | | | |
|---|---|---|---|---|---|---|
| ++, strong positive, +, Positive, +/-, weak positive or negative, -, negative | | | | | | |

## Claims

1. A construct comprising a cancer-homing peptide, wherein the cancer-homing peptide is selected from a linear pentapeptide CREKA (SEQ ID NO:1), AREKA (SEQ ID NO:23) or CREKA_{D} (SEQ ID NO:7), and a cyclic nonapeptide CPGPEGAGC (SEQ ID NO:2), wherein the two cysteines are cyclized, an optional linker and a cell-penetrating peptide coupled to an anticancer drug and/or a diagnostic agent.

2. The construct according to claim 1, wherein the optional linker is an amino acid or peptide.

3. The construct according to claim 1, wherein the cell-penetrating peptide is selected from the following peptide sequences
RQIKIWFQNRRMKWKK (SEQ ID NO:3)
GRKKRRQRRRPPQ (SEQ ID NO:4)
DAATATRGRSAASRPTERPRAPARSASRPRRVD (SEQ ID NO:5)
LUILRRRIRKQAHAHSKamide (SEQ ID NO:6)
RVIRVWFQNKRCKDKKamide (SEQ ID NO:7)
LGTYTQDFNKFHTFPQTAIGVGAP (SEQ ID NO:8)
LLGDFFRKSKEKIGKEFKRIVQRIKDFLRNLVPRTES (SEQ ID NO:9)
MANLG YWLLALFVTMWTDVGLCKKRPKPamide (SEQ ID NO: 10)
GWTLNSAGYLLGKINLKALAALAKKILamide (SEQ ID NO:11)
AGYLLG KINLKALAALAKKILamide (SEQ ID NO:12)
RRRRRRRRRRR (SEQ ID NO:13)
KLALKLALKALKAALKLAamide (SEQ ID NO: 14)
KETWWETWWTEWSQPKKKRKV (SEQ ID NO: 15)
KETWFETWFTEWSQPKKKRKV (SEQ ID NO:16)
GALFLGWLGAAGSTMGAPKKKRKV (SEQ ID NO: 17)
WEAKLAKALAKALAKHLAKALAKALKACEA (SEQ ID NO:18)
GLFKALLKLLKSLWKLLLKA (SEQ ID NO:19), and
GLFRALLRLLRSLWRLLLRA (SEQ ID NO-20).

4. The construct according to claim 1, wherein the anticancer drug is selected from alkylating agents, antimetabolites and cytotoxic antibiotics.

5. The construct according to claim 4, wherein the alkylating agent, is 4-[4-Bis(2-chloroethyl)amino)phenyl]butyric acid (chlorambucil) or 3-[4-(Bis(2-chioroethyl)amino)-phenyl]-L-alanine (Melphalan), the antimetabolite is /V-[4-(Λ/-(2,4-Diamino-6-pteridinyl-methyl)methylamino)-benzoyl]-L-glutamic acid (Methotrexate) and the cytotoxic antibiotic is (8S, 10S)-10-[(3-Amino-2,3,6-trideoxy-α-L-/yxo-hexopyranosyl)oxy]-8-glycoloyl-7,8,9,10-tetrahydro-6,8,11-trihydroxy-1-methoxy-5,12-naphthacenedione (Doxorubicin).

6. The construct according to claim 1, wherein the diagnostic agent is a fluorescent label.

7. The construct according to claim 6, wherein the diagnostic agent is a fluoresceinyl label.

8. The construct according to claim 1, wherein the construct contains the peptide CPGPEGAGCLLIILRRRIRKQAHAHSK-amide (SEQ ID NO:21).

9. The construct according to claim 1, wherein the construct contains peptide CREKALLIILRRRIRKQAHAHSK-amide (SEQ ID NO:22).

10. A method of delivering an anticancer drug and/or a diagnostic agent into a cancer cell comprising administration of a construct according to any one of claims 1-9 in vitro.

11. A construct according to any one of claims 1-9 for use as a medicament.

12. A construct according to any one of claims 1-9 for use in treatment of cancer.

13. A construct according to any one of claims 1-9 for use in diagnosing of cancer.

## Patentansprüche

1. Konstrukt, umfassend ein Krebs-Homing-Peptid, wobei das Krebs-Homing-Peptid unter einem linearen Pentapeptid CREKA (SEQ ID NO:1), AREKA (SEQ ID NO:23) oder CREKA_{D} (SEQ ID NO:27) und einem cyclischen Nonapeptid CPGPEGAGC (SEQ ID NO:2) ausgewählt ist, wobei die zwei Cysteine cyclisiert sind, einen optionalen Linker und ein zellpenetrierendes Peptid in Kopplung mit einem Arzneistoff gegen Krebs und/oder einem Diagnostikum.

2. Konstrukt nach Anspruch 1, wobei es sich bei dem optionalen Linker um eine Aminosäure oder ein Peptid handelt.

3. Konstrukt nach Anspruch 1, wobei das zellpenetrierende Peptid unter den folgenden Peptidsequenzen ausgewählt ist:
RQIKIWFQNRRMKWKK (SEQ ID NO:3)
GRKKRRQRRRPPQ (SEQ ID NO:4)
DAATATRGRSAASRPTERPRAPARSASRPRRVD (SEQ ID NO:5)
LUILRRRIRKQAHAHSK-Amid (SEQ ID NO:6)
RVIRVWFQNKRCKDKK-Amid (SEQ ID NO:7) LGTYTQDFNKFHTFPQTAIGVGAP (SEQ ID NO:8)
LLGDFFRKSKEKIGKEFKRIVQRIKDFLRNLVPRTES (SEQ ID NO:9)
MANLGYWLLALFVTMWTDVGLCKKRPKP-Amid (SEQ ID NO:10)
GWTLNSAGYLLGKINLKALAALAKKIL-Amid (SEQ ID NO:11)
AGYLLGKINLKALAALAKKIL-Amid (SEQ ID NO:12)
RRRRRRRRRRR (SEQ ID NO:13)
KLALKLALKALKAALKLA-Amid (SEQ ID NO:14)
KETWWETWWTEWSQPKKKRKV (SEQ ID NO:15)
KETWFETWFTEWSQPKKKRKV (SEQ ID NO:16)
GALFLGWLGAAGSTMGAPKKKRKV (SEQ ID NO:17)
WEAKLAKALAKALAKHLAKALAKALKACEA (SEQ ID NO:18)
GLFKALLKLLKSLWKLLLKA (SEQ ID NO:19) und
GLFRALLRLLRSLWRLLLRA (SEQ ID NO:20).

4. Konstrukt nach Anspruch 1, wobei der Arzneistoff gegen Krebs unter Alkylierungsmitteln, Antimetaboliten und zytotoxischen Antibiotika ausgewählt ist.

5. Konstrukt nach Anspruch 4, wobei es sich bei dem Alkylierungsmittel um 4-[4-Bis(2-chlorethyl)amino)phenyl]buttersäure (Chlorambucil) oder um 3-[4-(Bis-(2-chlorethyl)amino)phenyl]-L-alanin (Melphalan) handelt, bei dem Anitmetaboliten um N-[4-(N-(2,4-Diamino-6-pteridinylmethyl)methylamino)benzoyl]-L-glutaminsäure (Methotrexat) handelt und bei dem zytotoxischen Antibiotikum um (8S,10S)-10-[(3-Amino-2,3,6-trideoxy-α-L-/yxo-hexopyranosyl)oxy]-8-glycoloyl-7,8,9,10-tetrahydro-6,8,11-trihydroxy-1-methoxy-5,12-naphthacendion (Doxorubicin) handelt.

6. Konstrukt nach Anspruch 1, wobei es sich bei dem Diagnostikum um einen Fluoreszenzmarker handelt.

7. Konstrukt nach Anspruch 6, wobei es sich bei dem Diagnostikum um einen Fluoreszeinylmarker handelt.

8. Konstrukt nach Anspruch 1, wobei das Konstrukt das Peptid CPGPEGAGCLLIILRRRIRKQAHAHSK-Amid (SEQ ID NO:21) enthält.

9. Konstrukt nach Anspruch 1, wobei das Konstrukt das Peptid CREKALLIILRRRIRKQAHAHSK-Amid (SEQ ID NO:22) enthält.

10. Verfahren für die Abgabe eines Arzneistoffs gegen Krebs und/oder eines Diagnostikums in eine Krebszelle, bei dem man ein Konstrukt nach einem der Ansprüche 1-9 in vitro verabreicht.

11. Konstrukt nach einem der Ansprüche 1-9 für die Verwendung als Arzneimittel.

12. Konstrukt nach einem der Ansprüche 1-9 für die Verwendung bei der Behandlung von Krebs.

13. Konstrukt nach einem der Ansprüche 1-9 für die Verwendung bei der Diagnose von Krebs.

## Revendications

1. Construction comprenant un peptide de localisation du cancer, dans laquelle le peptide de localisation du cancer est choisi parmi un pentapeptide linéaire CREKA (SEQ ID NO:1), AREKA (SEQ ID NO:23) ou CREKA_{D} (SEQ ID NO:27), et un nonapeptide cyclique CPGPEGAGC (SEQ ID NO:2), où les deux cystéines sont cyclisées, un lieur éventuel et un peptide pénétrant dans la cellule (cell-penetrating peptide) couplé à un médicament anticancéreux et/ou à un agent diagnostique.

2. Construction selon la revendication 1, dans laquelle le lieur éventuel est un acide aminé ou un peptide.

3. Construction selon la revendication 1, dans laquelle le peptide pénétrant dans la cellule est choisi parmi les séquences peptidiques suivantes
RQIKIWFQNRRMKWKK (SEQ ID NO:3)
GRKKRRQRRRPPQ (SEQ ID NO:4)
DAATATRGRSAASRPTERPRAPARSASRPRRVD (SEQ ID NO:5)
LUILRRRIRKQAHAHSK-amide (SEQ ID NO:6)
RVIRVWFQNKRCKDKK-amide (SEQ ID NO:7)
LGTYTQDFNKFHTFPQTAIGVGAP (SEQ ID NO:8)
LLGDFFRKSKEKIGKEFKRIVQRIKDFLRNLVPRTES (SEQ ID NO:9)
MANLGYWLLALFVTMWTDVGLCKKRPKP-amide (SEQ ID NO:10)
GWTLNSAGYLLGKINLKALAALAKKIL-amide (SEQ ID NO:11)
AGYLLGKINLKALAALAKKIL-amide (SEQ ID NO:12)
RRRRRRRRRRR (SEQ ID NO:13)
KLALKLALKALKAALKLA-amide (SEQ ID NO:14)
KETWWETWWTEWSQPKKKRKV (SEQ ID NO:15)
KETWFETWFTEWSQPKKKRKV (SEQ ID NO:16)
GALFLGWLGAAGSTMGAPKKKRKV (SEQ ID NO:17)
WEAKLAKALAKALAKHLAKALAKALKACEA (SEQ ID NO:18)
GLFKALLKLLKSLWKLLLKA (SEQ ID NO:19), et
GLFRALLRLLRSLWRLLLRA (SEQ ID NO:20).

4. Construction selon la revendication 1, dans laquelle le médicament anticancéreux est choisi parmi des agents d'alkylation, des antimétabolites et des antibiotiques cytotoxiques.

5. Construction selon la revendication 4, dans laquelle l'agent d'alkylation est l'acide 4-[4-bis(2-chloroéthyl)amino)phényl]butyrique (chlorambucil) ou la 3-[4-(bis(2-chloroéthyl)amino)-phényl]-L-alanine (Melphalan), l'antimétabolite est l'acide N-[4-N-(2,4-diamino-6-ptéridinylméthyl)méthylamino)-benzoyl]-L-glutamique (Methotrexate) et l'antibiotique cytotoxique est la (8S,10S)-10-[(3-amino-2,3,6-tridésoxy-α-L-lyxo-hexopyrannosyl)oxy]-8-glycoloyl-7,8,9,10-tétrahydro-6,8,11-trihydroxy-1-méthoxy-5,12-naphtacènedione (Doxorubicin).

6. Construction selon la revendication 1, dans laquelle l'agent diagnostique est un marqueur fluorescent.

7. Construction selon la revendication 6, dans laquelle l'agent diagnostique est un marqueur fluorescéinyle.

8. Construction selon la revendication 1, dans laquelle la construction contient le peptide CPGPEGAGCLLIILRRRIRKQAHAHSK-amide (SEQ ID NO:21).

9. Construction selon la revendication 1, dans laquelle la construction contient le peptide CREKALLIILRRRIRKQAHAHSK-amide (SEQ ID NO:22).

10. Méthode pour administrer un médicament anticancéreux et/ou un agent diagnostique dans une cellule cancéreuse comprenant l'administration d'une construction selon l'une quelconque des revendications 1-9 in vitro.

11. Construction selon l'une quelconque des revendications 1-9 destinée à une utilisation comme médicament.

12. Construction selon l'une quelconque des revendications 1-9 destinée au traitement du cancer

13. Construction selon l'une quelconque des revendications 1-9 destinée au diagnostic du cancer.


### SEQUENCE LISTING

<110> CePep III AB
   Burnham Institute for Medical Research
<120> Chimeric constructs between cancer-homing peptides and cell-penetrating peptides coupled to anticancer drugs and/or diagnostic agent/agents
<130> P1101405PCT00
<150> US60/783,396
   <151> 2006-03-20
<160> 27
<170> PatentIn version 3.3
<210> 1
   <211> 5
   <212> PRT
   <213> Artificial sequence
<220>
   <223> Peptide - CREKA - G68
<400> 1
   Cys Arg Glu Lys Ala
   1 5
<210> 2
   <211> 9
   <212> PRT
   <213> Artificial sequence
<220>
   <223> Peptide - cyclic PEGA - M914
<400> 2
   Cys Pro Gly Pro Glu Gly Ala Gly Cys
   1 5
<210> 3
   <211> 16
   <212> PRT
   <213> Drosophila melanogaster
<220>
   <221> Peptide
   <222> (1)..(16)
   <223> Penetratin
400> 3
<210> 4
   <211> 13
   <212> PRT
   <213> Human immunodeficiency virus type 1
<220>
   <221> Peptide
   <222> (1)..(13)
   <223> Tat (48-60)
<400> 4
   Gly Arg Lys Lys Arg Arg Gln Arg Arg Arg Pro Pro Gln
   1 5 10
<210> 5
   <211> 33
   <212> PRT
   <213> herpes simplex virus type 1
<220>
   <221> Peptide
   <222> (1)..(33)
   <223> VP22
<400> 5
   Asp Ala Ala Thr Ala Thr Arg Gly Arg Ser Ala Ala Ser Arg Pro Thr
   1 5 10 15
   Glu Arg Pro Arg Ala Pro Ala Arg Ser Ala Ser Arg Pro Arg Arg Val
   20 25 30
   Asp
<210> 6
   <211> 18
   <212> PRT
   <213> Murinae gen. sp.
<220>
   <221> peptide_derived_from_murine_vascular_endothelial_cadherin
   <222> (1)..(18)
   <223> pVEC
<400> 6
   Leu Leu Ile Ile Leu Arg Arg Arg Ile Arg Lys Gln Ala His Ala His
   1 5 10 15
   Ser Lys
<210> 7
   <211> 16
   <212> PRT
   <213> Rattus sp.
<220>
   <221> Peptide
   <222> (1)..(16)
   <223> pISL
<400> 7
   Arg Val Ile Arg Val Trp Phe Gln Asn Lys Arg Cys Lys Asp Lys Lys
   1 5 10 15
<210> 8
   <211> 24
   <212> PRT
   <213> Homo sapiens
<220>
   <221> Peptide
   <222> (1)..(24)
   <223> hCT(9-32) derived peptide
<400> 8
   Leu Gly Thr Tyr Thr Gln Asp Phe Asn Lys Phe His Thr Phe Pro Gln
   1 5 10 15
   Thr Ala Ile Gly Val Gly Ala Pro
   20
<210> 9
   <211> 37
   <212> PRT
   <213> Homo sapiens
<220>
   <221> Peptide
   <222> (1)..(37)
   <223> LL-37
<400> 9
   Leu Leu Gly Asp Phe Phe Arg Lys Ser Lys Glu Lys Ile Gly Lys Glu
   1 5 10 15
   Phe Lys Arg Ile Val Gln Arg Ile Lys Asp Phe Leu Arg Asn Leu Val
   20 25 30
   Pro Arg Thr Glu Ser
   35
<210> 10
   <211> 28
   <212> PRT
   <213> Mus sp.
<220>
   <221> Peptide
   <222> (1)..(28)
   <223> Mouse PrP (1-28)
<400> 10
   Met Ala Asn Leu Gly Tyr Trp Leu Leu Ala Leu Phe Val Thr Met Trp
   1 5 10 15
   Thr Asp Val Gly Leu Cys Lys Lys Arg Pro Lys Pro
   20 25
<210> 11
   <211> 27
   <212> PRT
   <213> Artificial sequence
<220>
   <223> Peptide - Transportan (TP)
<400> 11
   Gly Trp Thr Leu Asn Ser Ala Gly Tyr Leu Leu Gly Lys Ile Asn Leu
   1 5 10 15
   Lys Ala Leu Ala Ala Leu Ala Lys Lys Ile Leu
   20 25
<210> 12
   <211> 21
   <212> PRT
   <213> Artificial seqence
<220>
   <223> Peptide - TP10
<400> 12
   Ala Gly Tyr Leu Leu Gly Lys Ile Asn Leu Lys Ala Leu Ala Ala Leu
   1 5 10 15
   Ala Lys Lys Ile Leu
   20
<210> 13
   <211> 11
   <212> PRT
   <213> Artificial seqence
<220>
   <223> Peptide - Arg11
<400> 13
   Arg Arg Arg Arg Arg Arg Arg Arg Arg Arg Arg
   1 5 10
<210> 14
   <211> 18
   <212> PRT
   <213> Artificial seqence
<220>
   <223> Peptide - MAP
<400> 14
   Lys Leu Ala Leu Lys Leu Ala Leu Lys Ala Leu Lys Ala Ala Leu Lys
   1 5 10 15
   Leu Ala
<210> 15
   <211> 21
   <212> PRT
   <213> Artificial seqence
<220>
   <223> Peptide - Pep-1
<400> 15
   Lys Glu Thr Trp Trp Glu Thr Trp Trp Thr Glu Trp Ser Gln Pro Lys
   1 5 10 15
   Lys Lys Arg Lys Val
   20
<210> 16
   <211> 21
   <212> PRT
   <213> Artificial sequence
<220>
   <223> Peptide - Pep-2
<400> 16
   Lys Glu Thr Trp Phe Glu Thr Trp Phe Thr Glu Trp Ser Gln Pro Lys
   1 5 10 15
   Lys Lys Arg Lys Val
   20
<210> 17
   <211> 24
   <212> PRT
   <213> Artificial sequence
<220>
   <223> Peptide - MPG
<400> 17
   Gly Ala Leu Phe Leu Gly Trp Leu Gly Ala Ala Gly Ser Thr Met Gly
   1 5 10 15
   Ala Pro Lys Lys Lys Arg Lys Val
   20
<210> 18
   <211> 30
   <212> PRT
   <213> Artificial sequence
<220>
   <223> Peptide - KALA
<400> 18
   Trp Glu Ala Lys Leu Ala Lys Ala Leu Ala Lys Ala Leu Ala Lys His
   1 5 10 15
   Leu Ala Lys Ala Leu Ala Lys Ala Leu Lys Ala Cys Glu Ala
   20 25 30
<210> 19
   <211> 20
   <212> PRT
   <213> Artificial sequence
<220>
   <223> Peptide - ppTG1
<400> 19
   Gly Leu Phe Lys Ala Leu Leu Lys Leu Leu Lys Ser Leu Trp Lys Leu
   1 5 10 15
   Leu Leu Lys Ala
   20
<210> 20
   <211> 20
   <212> PRT
   <213> Artificial sequence
<220>
   <223> Peptide - ppTG20
<400> 20
   Gly Leu Phe Arg Ala Leu Leu Arg Leu Leu Arg Ser Leu Trp Arg Leu
   1 5 10 15
   Leu Leu Arg Ala
   20
<210> 21
   <211> 27
   <212> PRT
   <213> Artificial sequence
<220>
   <223> Chimeric peptide - cyclic PEGA-pVEC
<400> 21
   Cys Pro Gly Pro Glu Gly Ala Gly Cys Leu Leu Ile Ile Leu Arg Arg
   1 5 10 15
   Arg Ile Arg Lys Gln Ala His Ala His Ser Lys
   20 25
<210> 22
   <211> 23
   <212> PRT
   <213> Artificial sequence
<220>
   <223> Chimeric peptide - CREKA-pVEC
<400> 22
   Cys Arg Glu Lys Ala Leu Leu Ile Ile Leu Arg Arg Arg Ile Arg Lys
   1 5 10 15
   Gln Ala His Ala His Ser Lys
   20
<210> 23
   <211> 5
   <212> PRT
   <213> Artificial sequence
<220>
   <223> Peptide - Ala1
<400> 23
   Ala Arg Glu Lys Ala
   1 5
<210> 24
   <211> 5
   <212> PRT
   <213> Artificial sequence
<220>
   <223> Peptide - Ala2
<400> 24
   Cys Ala Glu Lys Ala
   1 5
<210> 25
   <211> 5
   <212> PRT
   <213> Artificial sequence
<220>
   <223> Peptide - Ala3
<400> 25
   Cys Arg Ala Lys Ala
   1 5
<210> 26
   <211> 5
   <212> PRT
   <213> Artificial sequence
<220>
   <223> Peptide - Ala4
<400> 26
   Cys Arg Glu Ala Ala
   1 5
<210> 27
   <211> 5
   <212> PRT
   <213> Artificial sequence
<220>
   <223> Peptide - Ala5
<220>
   <221> MOD_RES
   <222> (5)..(5)
   <223> D- amino acid
<400> 27
   Cys Arg Glu Lys Ala
   1 5